# EUROPEAN PATENT APPLICATION

(11) **EP 2 583 671 A1**
(43) Date of publication of application: **24.04.2013**
(21) Application number: 11794970.1
(22) Date of filing: 14.06.2011
(51) Int. Cl.: A61K 9/107, A61K 47/36, A61K 47/34, A61K 47/38, A61K 47/32

(54) **COLLOIDAL NANOSCALE CARRIERS FOR ACTIVE HYDROPHILIC SUBSTANCES AND METHOD FOR PRODUCING SAME**

(30) Priority: 15.06.2010 BR PI1001959
(71) Applicant: Instituto de Pesquisas Tecnologicas do Estado de São Paulo S.A. IPT, 05508-901 Sao Paulo, SP (BR); Universidade De São Paulo-USP, 05508-900 São Paulo (BR); Fundação de Amparo à Pesquisa do Estado de São Paulo - FAPESP, 05468-901 São Paulo (BR)
(72) Inventor: NETO PEREIRA CERIZE, Natália, 04538-110 São Paulo (BR); MARIM DE OLIVEIRA, Adriano, 05017-110 São Paulo (BR); RÉ, Maria Inês, 05587-120 São Paulo (BR); TEDESCO, Antônio Cláudio, 14040-901 Ribeirão Preto (BR)
(74) Representative: De Hoop, Eric
(86) International application number: PCT/BR2011/000185
(87) International publication number: WO 2011/156880

(57) **Abstract**

The invention "colloidal nanoscale carriers for active hydrophilic substances and method for producing same" pertains to the field of medical, odontological or hygiene preparations, and is characterized by structures formed by hydrophilic polymers that contain active hydrophilic substances coated with a non-hydrophilic phase and surfactants with affinity for the components, forming an invert emulsion that allows the incorporation and controlled delivery of active hydrophilic substances, conferring properties such as protection against degradation processes, improvement of compatibility with the other components of the formulation in the final product, increase in the availability and/or bioavailability of the active substance in the medium of interest (including improvements in permeation processes in biological materials, reduction of the exposure and volatilization of the active substance in the medium) and controlled release of the active substance(s). The nanoscale carrier obtained by this method, called colloidal nanoscale carrier (NC), can be used in various fields, such as the pharmaceutical field (including dermatology), cosmetics, personal hygiene products, veterinary medicine, agrochemicals and fertilizers, the food industry and the like. The invention proposes a kinetically stable system with an effective nanoscale structure that consists of nanoscale carriers formed by polymers emulsified in a non-aqueous medium in the presence of a surfactant with affinity for the two phases (the dispersion medium and the encapsulating agent). This system is obtained by nanoemulsification of an aqueous phase of hydrophilic polymers emulsified in a non-hydrophilic (lipophilic or silophilic) phase that contains the surfactants, and is characterized by the implementation of two concepts that encompass the generation of an invert nanoscale emulsion and of polymer nanoparticles. The formulation has the novel technical effect of providing a polymer excipient with a nanoscale structure for delivering hydrophilic molecules suspended in a non-hydrophilic phase, which allows controlling the size of the nanoscale particles and modulating colloidal stability by means of process parameters.

## Description

### FIELD OF THE INVENTION

The present invention pertains to the area of preparations for medical purposes (including pharmaceutical, cosmetic and personal care) and also apply a chemical process that involves the chemistry of colloids and fields characterized by technical aspects, specifically nanotechnology, working by means of a process for obtaining a polymeric colloidal nanocarrier allowing incorporation and serving as a controlled delivery system for actives, conferring hydrophilic properties such as protection against degradation, system stability, improved compatibility with the other constituents of the formulation in the final product, increase in the availability and / or bioavailability of the actives in the medium of interest (including process improvements in terms of permeation in the biological media, exposure reduction and volatilization of the actives to the said media) and controlled release actives. The nanocarrier obtained by this process can be applied in areas as diverse as pharmaceuticals (including dermatology), cosmetics, toiletries, veterinary goods, agrochemical and fertilizer, food and the like.

### OBJECTIVE OF THE INVENTION

A polymeric colloidal nanocarrier product enabling the incorporation and controlled placement of hydrophilic actives and its production process.

### PRIOR ART

In the rational development of a delivery system or appropriate vehicle that meets the criteria of quality, efficacy and safety, it is important to understand the physicochemical properties of the active, such as polymorphic forms, compatibility with other formulation components during processing and storage, system stability, as well as the route of administration (orally, topically, parenterally) and release form (immediate release, controlled release, sustained release). In this context, the pre-formulation study is essential for it covers the idealization of the formulation, identification of the characteristics of the active and excipients, verifying the stability under stress conditions (conditions of extreme pH and temperature) and compatibility studies.

The placement of drugs accurately and securely, at the right time, with controlled release and reaching the maximum therapeutic effect at the site of action remains a reference in the design and development of new drug delivery systems. The concept of site-specific release relies on the very idea of minimizing the risk-benefit parameter. The nanocarriers, in their various forms, have the possibility of providing endless opportunities in regard to drug delivery and therefore are increasingly studied in order to exploit the potential thereof (Mishra; Bhavesh, Sanjay, 2009).

However, success in formulating a site-specific nanocarrier is not only to reach the target, but also to convey the drug in its molecular form, keeping its pharmacological activity and allowing its interaction with the receptor. Factors such as loss of carrier for drug release or degradation, reduced absorption in the target, or reduced thermodynamic activity of active abduction of proteins can not be neglected, otherwise the systems can fail not reaching the site of action in sufficient quantities and release rate and diffusion of the drug below the optimal concentration, not promoting the required therapeutic effect (Ruenraroengsak, and Florence Cook, 2010).

The nanocarriers, due to the high surface area thereof, show improvements in pharmacokinetics and biodistribution of therapeutic agents and thus minimize toxicity for preferential accumulation at the site of action. May improve the solubility of hydrophobic compounds and making them suitable for parenteral administration and also increase the stability of a variety of therapeutic agents such as peptides, oligonucleotides, among others (Wu et al. To 2001; Arruebo et al. , 2007; (Mishra; Bhavesh, Sanjay, 2009), improve the bioavailability of the drug at the site of action and facilitate cellular internalization (Torchilin, 2009).

Moreover, as one of the key advantages of nanocarriers is their size, and any circumstance that alters its initial design in diameter can cause complications concerning specificity and likelihood of decreased ability to reach the target (Ruenraroengsak, Cook and Florence, 2010).

### Solid Lipid Nanoparticles and Nanoestructured Lipid carriers

The solid lipid nanoparticles (NLSs) are classified into the nanoscale (from 50 - 1000 nm) and were proposed, among others, as promising systems for topical application (Mühlen et al., 1998; GOYMANN-MULLER, 2004). They are formed by a single layer, unlike liposomes (phospholipid vesicles in bi-layers), which may (liposomes) form lamellar structures with one or several concentric membranes formed by lipid-water (LIMA-Kedor and Hackmann, 1994). The NLSs are composed of excipients well tolerated by the skin, and raw materials commonly used in pharmaceutical and cosmetic formulations can be employed in these systems (Muller et al. 2000). The substances used include triglycerides, glycerides, fatty acids (e.g. stearic acid) and waxes (eg cetyl palmitate). A new type of lipid nanoparticle using mixtures of solid and liquid lipids has been studied (nanostructured lipid carrier - CLN). The resulting lipid particle has a nanoparticulate solid structure with depressions formed by the liquid lipid (oil) (Muller et al. 2002). To prepare these systems techniques are used with ultrasound and high pressure homogenizers (either cold or hot processes), emulsification and solvent evaporation and microemulsification (MEHNERT and MADER, 2001). The CLNs have advantages such as the ability to protect against chemical decomposition of labile components, the possibility of controlled release of substances through the solid state of the lipid matrix, possibilities of forming a film on the skin and occlusive properties (Muller et al., 2002 ). Jennings and coworkers stand still, the small size of the nanoparticles, which have large surface area, facilitating the contact of the encapsulated substances to the *stratum corneum* and consequently the amount capable of penetrating the viable skin (Jennings et al. 2000; MAIA et al. 2000).

The NLSs have an occlusive effect more intense when compared with conventional emulsions or microparticles. The occlusion is based on forming a film after application to the skin by reducing transepidermal water loss (Wissing and Müller, 2001). With increasing water content in the skin, the symptoms of atopic dermatitis can be reduced, contributing to skin health. An increase of occlusivity can be checked when the NLSs are added to oil / water emulsions, increasing inclusive, the effect of hydration (Wissing and MULLER, 2002a). The extent of the occlusive properties is also dependent on factors such as particle size and lipid concentration (Wissing and Muller, 2002b).

The physicochemical stability of the carried substances in NLSs and CLNs can be totally different from non-carried ones (free form). The effect of formulation on the physicochemical aspect of the associated structure must be investigated individually, thereby enabling the development of formulations suitable for each case (LIM and Kim, 2002). For example, studies with retinol and coenzyme Q10 demonstrated that NLSs by incorporating these actives, the solid matrix decreased chemical degradation of these substances (Muller et al. 2000). Studies have shown further that the physical stability of these systems can be maintained when incorporated into vehicles suitable for topical administration. Thickening agents, humectants and surfactants contribute to stabilize the formulations and sustained release modulating substances from NLSs (Jennings et al. 2000; LIPPACHER et al. 2001).

### Double Emulsion, crystallizable

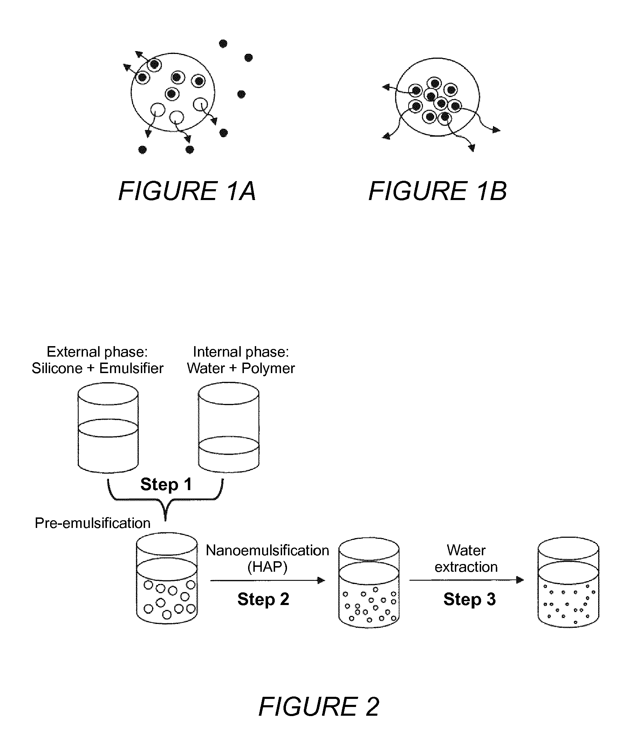

The crystallizable double emulsions form a kinetically stable system of microreservoiurs designed from the concept of the conventional dual emulsion (an internal aqueous phase emulsified in an oil phase and aqueous phase elsewhere reemulsified), in which case the membrane separating the two phases Aqueous consists of a lipid component solid at room temperature, as shown in Figure 1. The physicochemical nature of the constituent solid influences the time of destruction and encapsulation capacity of the system.

Several factors influence the stability of this type of system, eg, the osmotic pressure of the internal and external aqueous phases, interactions between the particles (surface charge of crystallizable oils, interactions spherical) surface properties and conformation acquired by the oily phase After cooling and rheological evolution of the oil phase among others (GUERY, J., 2006), however, the crystallizable double emulsions arrays are permeable to water and permeable to species can be encapsulated hydrophilic, whereas the lipid core is a solid state semipermeable membrane.

The osmotic conditions allow you to control precisely the properties of encapsulation and release active. Under iso-osmotic diffusion of the active is slow, whereas in the middle hypo-osmotic release is fast, followed by a disintegration of the material. The kinetics of the release process is controlled by the membrane organization in the solid state, initial distribution of the lipid matrix and the ability to contract or expand. These parameters can be adjusted by the choice of-crystallizable oils and also by changing the thermal history of composite material (GUERY, J., 2006).

### Nanoemulsions

Nanoemulsions are transparent or translucent systems within a range of 50 - 200 nm that are kinetically stable, however the long term stability (no apparent flocculation or coalescence) characterizes nanoemulsions as a differentiated system, with a certain thermodynamic stability (Tadros et al. 2004). The high colloidal stability of nanoemulsions can be understood by considering the stabilization spherical (using non-ionic emulsifiers and polymeric), in addition to being affected by the thickness of the emulsifier and the radius of the droplet. Furthermore, the droplet size in the nanoscale causes a large reduction in force of gravity and in this case the Brownian motion overcomes this force, preventing sedimentation or creaming during storage. The small size also prevents coalescence and flocculation processes, since the drops are non-deformable and inhibit variations in the surface. The film thickness of surfactant (relative to the droplet radius) prevents any weakening or rupture of liquids between the layers (Tadros et al. 2004).

These systems are very attractive for placement of topical products since the large surface area allows quick penetration of actives, due to the reduced size, the nanoemulsions may promote improved skin permeation of active can be prepared using lower concentrations of emulsifying microemulsions which and transparency and promote a sense of fluidity nice application (Tadros et al., 2004). Nanoemulsions containing plasmid DNA (Wu et al. 2001a), ceramides (YILMAZ and Borchert, 2005), oil of citronella (SAKULKU et al. 2009), camphor, menthol and methyl salicylate (MOU et al. 2,008) were reported for topical application.

### Inverse nanoemulsion (water-in-oil)

Considering the pharmaceutical applications of topical dermatological for placement of nanostructures, the proposal to produce a nanoemulsion inverse (water in oil), at the nanoscale, would be an ideal candidate as a nanocarrier for active hydrophilic, based on the perspective of the molecule remain in phase internal be possible to work the pH to improve stability, reduce the possibility of degradation and improve its bioavailability. Literature report showed that the carried macromolecules in inverse emulsions are possibly transported via transfollicularly or via the transepidermal is achieved by disruptions in the permeability of the *stratum corneum* caused by surfactants (Wu et al. 2001b). In this work, Wu and colleagues (2001) showed that the emulsions with HLB value (hydrophile-lipophile balance) compatible with the standard tallow (produced by the sebaceous glands of the skin) may be carrying hydrophilic molecules due to a facilitated co-transport mediated primarily via transfollicularly.

The limitations of this process are connected with the mixing step and emulsifying phase, which is usually carried out at high temperatures (above room temperature). Since the degradation of active in solution is accelerated with increasing temperature, it is necessary to work with materials and liquids that do not require heating in the process of formation of the emulsion.

### Anhydrous nanoemulsion

Another delivery strategy considered novel among the systems mentioned above, would be the development of a nanoemulsion that is anhydrous. This is one nanostructured system, which uses a non-aqueous solvent for solubilization of hydrophilic active that may be degraded in aqueous phase. Such systems, which may replace the conventional emulsions where the presence of water must be avoided, have been used for the preparation of nanoparticles and as templates for the formation of microstructures silicate (SUITTHIMEATHEGORN et al. 2005).

An interesting phenomenon in non-aqueous systems is the formation of the ion pair to form structures with different physical characteristics of the ions. The ion pair facilitates the permeation of the ionized active through hydrophobic membranes, based on the hypothesis that these actives may obtain a certain electrical neutrality and lipophilicity via ion-pair formation. The ion pairs formed in non-aqueous systems can permeate through the membrane pores and mechanisms partition, which illustrates the possibility of facilitating the permeation of active ionizable hydrophobic membrane (Lee et al. 1988).

The main drawbacks of these systems, however, are configured on the solubility of the hydrophilic agents into a nonaqueous medium, the choice of nonaqueous medium, and also the process for obtaining these systems in the nanometer range.

### Polymeric nanoparticles

The development of nanoparticles with polymeric coating is also configured as an interesting alternative in order to encapsulate hydrophilic molecules stability, encapsulation efficiency and increased bioavailability. In this case, one strategy is to encapsulation using a hydrophilic polymer as a coating agent or matrix forming through the emulsification process and diffusion of solvent (NAGARWAL et al. 2009). However, in a formulation in which the nanoparticles remain suspended, it is important to ensure that active not migrate into the dispersing medium, which could result in their degradation and also the selection of the process for obtaining polymeric nanoparticles should ensure the viability of active with regard to use of heat and solvents.

### SUMMARY OF THE INVENTION

This "COLLOIDAL NANOCARRIERS FOR HYDROPHILIC ACTIVES AND THEIR PRODUCTION PROCESS" invention describes a system kinetically stable and effective in the nanostructure, that is to nanocarriers formed of polymers emulsified in a non-aqueous medium in the presence of a surfactant having attraction between the two phases (half dispersing agent and encapsulant). This new system is obtained by the nanoemulsification of an aqueous phase containing hydrophilic polymers, non-emulsified in a hydrophilic phase (lipophilic or silophilic) containing the surfactant, especially by application of two concepts that comprise the generation of a nanoemulsion and inverse polymeric nanoparticles. The formulation in the grounded concepts of nanoemulsion and reverse polymeric nanoparticles generates the new technical effect of a nanostructured polymeric carrier for serving hydrophilic molecules suspended in no hydrophilic phase, capable of controlling particle size in the nanometer range and modulating the colloidal stability by means of the process parameters.

The achievement of this system, referred to as Colloidal nanocarriers (NCs) is possible with the use of a process combining the steps of nanoemulsification with high pressure homogenization and partial extraction of the water content of the internal phase, the latter being regarded as a phase factor in the stability of the formulation by enabling modular system characteristics.

The NCs exemplified herein resulted in a formulation with suitable characteristics for the placement of hydrophilic molecules, reproducing the physical properties of the system which agrees with placebo in the viscosity, average particle size, morphology and stability, yet having high encapsulation efficiency and profile Controlled release / sustained. The behavior of systems containing hydrophilic allows models to better understand the formation mechanism of the NCs, the active form of interaction with the polymer matrix and identify the physical differences (especially morphology) and chemical (encapsulation efficiency and release profile) for two models tested.

With the invention of these new systems NCs are promising for serving active hydrophilic, with the potential to stabilize them in a medium with reduced amount of water, improving several application properties. This new carrier system contributes to innovation and creation of new forms of administration and delivery of active agents of interest in various areas such as pharmaceuticals (including dermatology), cosmetics, toiletries, veterinary, agrochemical and food.

### BRIEF DESCRIPTION OF DRAWINGS

Figure 1. Is a sectional schematic drawing showing the advantages of emulsions in relation to the double crystallizable liquid emulsions pairs (adapted from Guery, 2006) wherein Fig 1A shows dual liquid emulsion showing the coalescence of droplets of the internal phase at the interface of cells with diffusion through liquid oily phase, and Fig 1B shows crystallizable double emulsion showing no coalescence of the droplets of the internal phase at the interface, with reduced permeability through the solid membrane.
Figure 2. Is a schematic drawing of the Manufacturing Process of NCs, showing the three steps of the process.
Figure 3. Is a scanning electron microscopy of the product of Example 1 obtained by FEG-SEM, which shows the formation of nanocarriers with low polydispersity of spherical morphology and smooth and regular surface. The characteristics of the photomicrograph are marked thereon.
Figure 4. Figure 4A shows the graphic profile of extracting water internal phase of the NCs of PVP as a function of particle size, i.e. the particle size variation as a function of the water content present in nanocarriers and Figure 4B shows the graph of the NCs backscatter-based PVP 3 hours of water extraction process, indicating no change in backscatering and consequent stability of the sample for 7 days, all referring to a NCs obtained in EXAMPLE 2.
Figure 5. Figure 5A shows photomicrograph of a NCs based on chitosan obtained by FEG-SEM, where the characteristics of the photomicrograph are in itself, Figure 5B shows a graph of profile extraction water internal phase of the chitosan NCs as a function of particle size, and Figure 5C shows a graph of the kinetic stability of NCs based on chitosan with 3 and 5 hours of water extraction process, all referring to NCs obtained in EXAMPLE 3.
Figure 6. Figure 6A shows photomicrograph of the starch-based NCs containing sodium salicylate as active hydrophilic model obtained by FEG-SEM, where the characteristics of the photomicrograph are in itself, and Figure 6B shows further enlarged photomicrograph of the NCs starch containing sodium salicylate as active hydrophilic model obtained by FEG-SEM, where the characteristics of the photomicrograph are on their own, all related to the a NC obtained in EXAMPLE 4.
Figure 7. Figure 7 shows a graph of the kinetic stability of the NCs, wherein the NCs referr to EXAMPLE 5 based on PVP containing Sana with 3 and 5 hours of water extraction process.

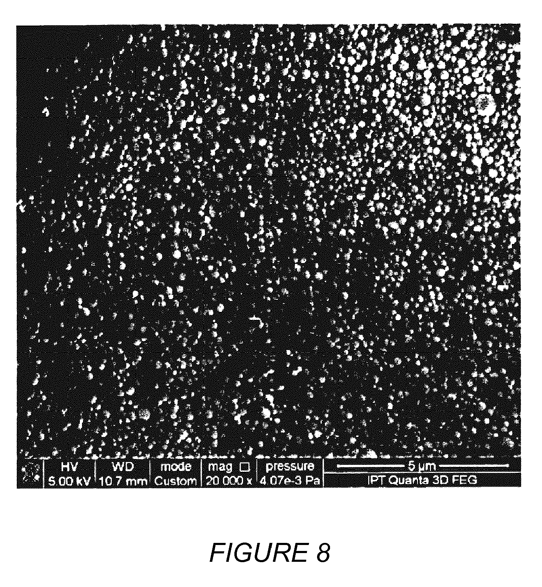

Figure 8. Figure 8 shows a photomicrograph of NCs, referring to Example 6, with starch-based Cyanocobalamin containing as active hydrophilic model obtained by FEG-SEM, wherein the characteristics of the photomicrograph are displayed thereon.
Figure 9. Figure 9 shows a graph of the release profile of sodium salicylate NCs starch-based relating to Examples 4 and 6.

### DETAILED DESCRIPTION OF THE INVENTION

The development of a system for delivering nanostructured actives, seeking improved stability and increased bioavailability, is a challenge of the science of colloids covering numerous applications, ranging from the placement of drugs with site-specific action, the skin permeation of hydrophilic compounds to cellular internalization of particles. Faced with these challenges many systems have been considered in recent years, but many have limitations with respect to an action potent and stable for a given application.

Nanoparticles have the advantage of having a solid matrix that holds the active component and may modulate their release. The solid state of these materials gives them a reduced permeability to containing species. Moreover, the solid particles lipid-based reduce any limitation due to toxicity problems. These materials are certainly effective for the encapsulation of hydrophobic molecules, but are limited to ensure vectoring hydrophilic species, or for any application requiring aqueous internal compartment.

The crystallizable double emulsions systems would also be very viable for the placement of hydrophilic active, having as limiting the warming issue during the preparation as well as polymeric nanoparticles.

The nanoemulsions have emerged as the ideal candidate for the application in question, but regarding the incorporation of hydrophilic molecules and possessing degradation in aqueous medium, there was a need for creating an inverse emulsion or even anhydrous, which avoided its degradation.

The nanocarrier discussed herein is thus resulting from the design of a new system kinetically stable and effective in the nanostructure. This new nanocarrier consists of structures formed by hydrophilic polymers containing hydrophilic active, surrounded by a hydrophilic phase and surfactants not having attraction for the components, forming an inverse emulsion formed by polymer emulsified in a non-aqueous medium in the presence of a surfactant which has attraction between the two phases (the dispersing medium and encapsulating agent). Employing hydrophilic polymers dissolved in the aqueous phase and emulsified in a non-aqueous phase containing the surfactant, this design combines both concepts covering the generation of a nanoemulsion and an inverse polymeric nanoparticles.

The new system is called Colloidal nanocarrier (NC) and consists of a polymeric colloidal nanocarrier that enables controlled placement and incorporation of active hydrophilic formulated in a non-aqueous external phase.

Some inventions have been patented using nanoparticles and carrier, however, mostly refers to the development and implementation of carriers for hydrophobic molecules, also using hydrophobic encapsulating matrix, eg, block copolymers, and still employing various processes, and in neither case is used in obtaining the inverse emulsion nanocarriers, this is basically the novelty and inventiveness of the COLLOIDAL NANOCARRIERS FOR HYDROPHILIC ACTIVES AND THEIR PRODUCTION PROCESS that are object of this patent application.

Document WO 2009123768 "Nanocarrier and Nanogel Compositions," describes a class of carriers in the nanometer range consisting of block copolymers suspended in an aqueous solvent or co-polymer (in a different way to the invention disclosed herein) associated with therapeutic agents hydrophobic character, and as examples tested were employed indomethacin, doxorubicin and budesonide, among others.

Another composition, presented in document WO 2007041206 "Drug Delivery nanocarriers Targeted by Phase Landscape" describes method of obtaining nanocarriers by employing amphiphilic molecules for encapsulation, particularly site-specific protein, using only the technique described complexation. The carrier is formed by a phase protein containing a protein that exhibits a peptide linker selected to bind specifically and selectively to a target site, which is released. However, the invention only claims specific proteins, including non-hydrophilic molecules or as polymer matrices employing nanocarrier.

Documents KR 100868724 "Method for Preparing Self-Aggregating Nanocarrier Particles Having Temperature Depending Property" and WO 2009123934 "Branched Multifunctional Nanoparticle Conjugates and their Use" also describe carrier nanoparticles for hydrophobic agents. In the first case (KR 100868724), the inventors use block copolymers that are temperature sensitive for the incorporation of drugs, and control of particle formation by means of temperature. The process involved comprises a polymerization step and employs sensitive polymers containing a group of poly (N, N-dimethylacrylamide), poly (N-isopropyl acrylamide) or mixture thereof. In the case of WO 2009/123934, the matrix is composed of branched polymers polyglycerol with specific action "mount and unmount" in vivo conditions, coupled with a hydrophobic agent. These systems are often employed in imaging and diagnostic procedures, such as in cancer models.

The WO 2009055794 document "Method and Compositions for Therapeutic Molecules Containing Polymer nanocarriers" describes a method and a composition nanocarriers formed by block copolymers of hydrophobic obtained by the double emulsion process for encapsulation and delivery of proteins. The application comprises the use of this filamentous and spherical nanoparticles carrier for diagnostics and therapeutics.

Document WO 2009141170 "Suitable Nanocarriers for Active Agents and Their Use" discloses a carrier body which has in its structure a grouping defined by formula amine as a residue. The invention relates to compounds such as carrier for nucleotides, nucleosides, oligonucleotides linear or circular single or double and oligomeric molecules (being all of these hydrophobic molecules), with a shell consisting of polyglycerol and / or derivatives, with the main field of interest being the silencing genes. The processes for the obtention of NON nanocarriers consists in reverse emulsion, such as the invention herein disclosed, but rather a conventional emulsion (oil / water) to the airing of hydrophobic actives.

The prior art work that presents some respects similar to the present invention is document U.S. 2009/0258078 "Antioxidant Polymer Nanocarriers for Use in Preventing Oxidative Injury" which is characterized by the presence of a carrier polymer for encapsulation of proteins prepared by homogenization temperature below zero, thus maintaining the enzyme activity. May be employed xenobiotic detoxifying enzymes and antioxidants, which are preserved from degradation of proteases, increasing their lifespan. One advantage is that the system is permeable to substrate and can exert its effect without release of the encapsulated enzyme.

The present invention "COLLOIDAL NANOCARRIERS FOR HYDROPHILIC ACTIVES AND THEIR PRODUCTION PROCESS" employs a nanocarrier for molecules hydrophilic properties (and not protein), suspended in a non-aqueous medium and obtained by a method that includes three stages: pre-emulsification, extraction and nanoemulsification of the internal phase (solvent), by means of a double-emulsion process. The nanocarrier formed for hydrophilic agents also protects against degradation and may protect including molecules susceptible to degradation in aqueous medium and non-enzymatic action as in document U.S. 2009/0258078. In addition, there is provided the controlled-release profile of the nanocarrier colloidal which can be modulated as a function of active agent employed, differently to the aforementioned invention, wherein the encapsulated protein remains coupled with the carrier to exert its effect, not being released.

The present invention "COLLOIDAL NANOCARRIERS FOR HYDROPHILIC ACTIVES AND THEIR PRODUCTION PROCESS" presents a polymeric nanocarrier system formation and structure well defined, and the production process developed specifically for the limitations found in all works presented here, which is the encapsulation Agents hydrophilic simply, in a single emulsification process (without the use of double-emulsion), and even using inert materials, biodegradable and even in some cases.

The manufacturing process of the NCs preparation involves three steps: Step 1 being the pre-emulsification step, whilst Step 2 and Step 3 are nanoemulsification and extraction of the internal phase, as shown in Figure 2, the NCs disclosed herein being obtained by emulsification of a hydrophilic polymers containing aqueous solution with the active principle of interest, such polymers are polysaccharides, protein of animal or vegetable origin, chitosan, gum (acacia gum, xanthan gum, guar gum, carrageenan gum, cashew gum, tara gum, tragacanth gum, karaya gum, gati gum), cellulose derivatives (carboxymethyl cellulose, carboxyethyl cellulose, etc.), polyvinylpyrrolidone, polyacrylates, polyacrylamides, polivinilcaprolactamas in a hydrophilic phase not containing emulsifying agents compatible with the specific hydrophilic phase not chosen. This phase cannot be made hydrophilic by both lipophilic or silophilic liquids. The emulsification process can be carried out by various conventional techniques, such as mechanical stirring, cowlles, ultraturrax, high-pressure homogenizers, ultrasound, or any other technique that will promote the emulsification of an aqueous phase in a nonaqueous environment.

Step 1. Formation of the pre-emulsion by dispersing the internal phase in the external phase under mechanical stirring and after complete addition, use of conventional stirrer.

In this first step is pre-emulsion formed between the inner and outer phase, the inner phase being composed of polymer and an aqueous solution containing an inorganic salt (water soluble salts) which function as co-stabilizer and the active hydrophilic, while the external phase contains the non-hydrophilic component and a specific emulsifier such as silicone-modified polioxydethylene (SF1540, Momentive ®) or other customary emulsifiers compatible.

The temperature employed in this step of the process may vary from 10 to 100 ° C, preferably 25.0 ° C. The mixture is held under stirring, which can vary from 100 to 22,000 rpm, preferably 1000 rpm and under atmospheric pressure. The salts used should be water soluble, preferably chlorides that are mono-or bivalent.

Step 2. Homogenizing the pre-emulsion formed in Step 1 in a system of high energy mix of disaggregation.

The use of high pressure homogenization must employ a minimum of one cycle of homogenization up to the amount required to achieve the desired particle size, generally below 20 cycles, the temperature employed in this step can vary from 10 to 100 ° C, preferably 25.0 ° C. The pressure equipment must be at least 10 bar and maximum capacity of the pressurizing device, preferably 900 bar.

Step 3. The resulting nanoemulsion is placed in a reactor with reduced pressure with controlled temperature and mild agitation was connected to a condenser for extraction of the internal aqueous phase and formation of NCs.

This step of extracting the solvent of the internal phase should be performed for at least 15 minutes to the time required for dehumidification desired, usually 5 hours, and the pressure applied may vary from 760 mmHg to 10 -7 mmHg, preferably 280 mmHg. The reactor temperature in this step can vary from 20 to 150 ° C, 50 ° C being the preferred temperature.

### EXAMPLES

To illustrate some embodiments of the invention and the potential application of NCs are examples employing different active hydrophilic, and the main characteristics of the products obtained, including the release profile and encapsulation efficiency.

The NCs were characterized as the water content, refractive index, mean particle diameter, polydispersity, viscosity, turbidity dynamics and morphology.

### EXAMPLE 1: Obtaining CN starch-based in silicone

In a 500 mL beaker was prepared a solution containing 140g of an emulsifier-based silicone-modified polioxydethylene (SF1540, Momentive ®) in the concentration dimethicone 3% m / m. Another solution was prepared by dissolution of 9 g of starch and 0.6 g NaCl in 51 g of deionized water. The aqueous starch solution was emulsified in the hydrophilic phase not under mechanical agitation of 1000 rpm. After this emulsification, the mixture was subjected to high pressure homogenization for five cycles at a pressure of 900 bar. Finally, the emulsion was brought to a jacketed glass reactor to effect the removal of water by distillation under vacuum, at pressure of 280 mmHg. The water removal was conducted for 3 hours and 5 hours in dehumidifying 50 ° C. The NCs were characterized as the average particle diameter (DP), polydispersity index (PI), residual water content (TA), viscosity (Target), turbidimetry and dynamic morphology. The results are shown in Table 1.

**Table 1: Results obtained with the product produced according to the experimental conditions of Example 1.**

| **DP (nm)** | | **IP** | | **TA (%)** | | **Visc** |
|---|---|---|---|---|---|---|
| 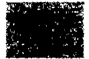 | *5h* | 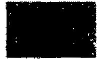 | *5h* | 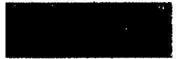 | *5h* | **(cP)** |
| 442 | 168 | 1,00 | 0,113 | 4,76 ± 0,350 | 0,087 ± 0,002 | 144 |

The results of PD, PI and AT reported in Table 1 refer to NCs obtained in times 3 hours and 5 hours of water extraction process and certify the ownership of nanometer carrier system, with relatively low polydispersity index, and the content of reduced water as a function of time. Furthermore, the system has generated characteristic of fluid, as can be seen by the low viscosity displayed.

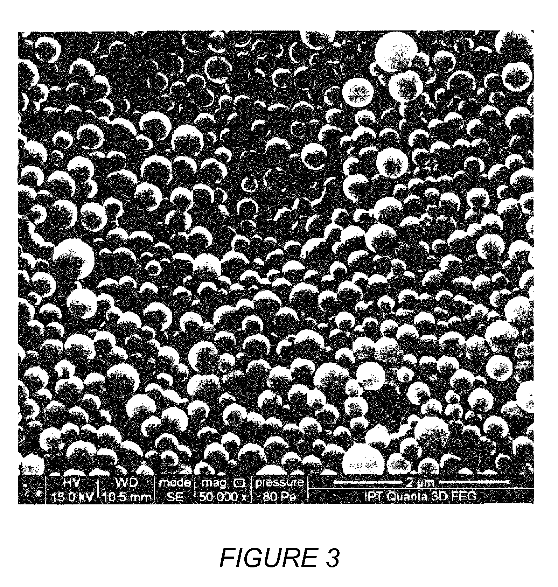

The morphology spherical and smooth surface, regular NC obtained can be seen in Figure 3.

### EXAMPLE 2: Obtaining NC-based polyvinylpyrrolidone (PVP) in silicone

In a 500 mL beaker was prepared 140 g of a solution containing an emulsifier to the silicone-modified polioxydethylene (SF1540, Momentive ®) in the concentration dimethicone 3% by mass. Another solution was prepared by dissolution of 9 g of PVP and 0.6 g NaCl in 51 g of deionized water. The water solution of PVP was emulsified in the hydrophilic phase not under mechanical agitation of 1000 rpm. After this emulsification, the mixture was subjected to high pressure homogenization with five cycles at 900 bar pressure. Finally, the emulsion was brought to a jacketed glass reactor to effect the removal of water by distillation under vacuum (280 mmHg). The water removal was conducted for 3 hours and 5 hours in case of temperature of 50 ° C. The NCs obtained in this example were characterized as the average particle diameter (DP), polydispersity index (PI), residual water content (TA), viscosity (Target), refractive index (RI), turbidimetry and dynamic reduction profile size as a function of water content. The results are shown in Table 2.

**Table 2: Results obtained with the product produced according to the experimental conditions of EXAMPLE 2.**

| **DP (nm)** | | **IP** | | **TA (%)** | | **Visc** | **IR** |
|---|---|---|---|---|---|---|---|
| 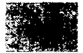 | ***5h*** | 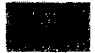 | ***5h*** | 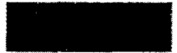 | ***5h*** | **(cP)** | **(25°)** |
| 157 | 176 | 0,438 | 0,023 | 5,513 ± 0,106 | 0,338 ± 0,004 | 135 | 1,401 |

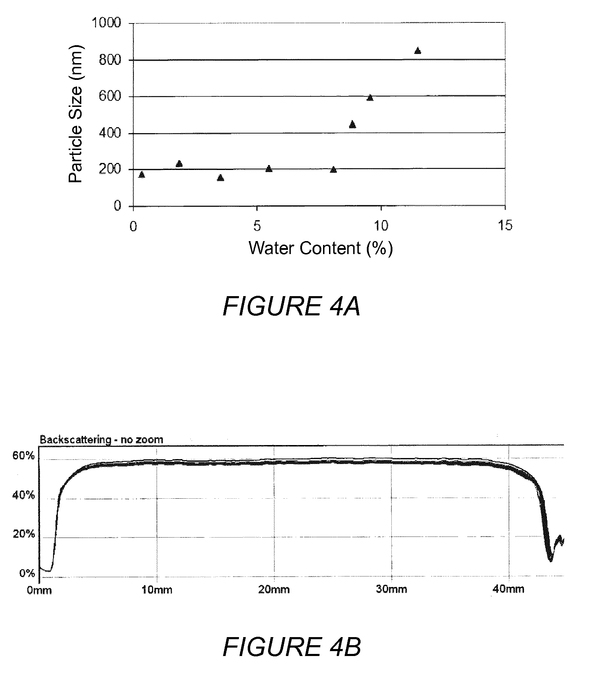

The results of NCs-based PVP were similar to those described for starch NCs shown in EXAMPLE 1. The values of PA and TA PI described in Table 2 refer to NCs obtained at times 3 hours and 5 hours of water extraction process and confirm the property of nanometer-carrier system consisting of PVP, with relatively low polydispersity and reduced water content as a function of time. Moreover, the generated system has a characteristic of fluid, as can be seen by the low viscosity displayed. It is emphasized that, controlling the step of extracting water from the internal phase, it is possible to modulate the particle size of the nanocarriers formed, reaching the level of desired diameter as shown in Figure 4A. The results show that it is necessary to obtain a given quantity of water to obtain nanostructured systems, from which no further significant variation occurs in size, which can interfere with the physical stability, which can be observed by turbidimetry dynamic as shown in Figure 4B . The NCs-based PVP obtained with 3 hours of dehumidification were analyzed for backscatter profile (by turbidimetry dynamic) for 7 days after preparation, and showed high physical stability.

### EXAMPLE 3: Obtaining CN chitosan-based in silicone

Obtaining the CN based on chitosan was performed in a 500 mL beaker was prepared where 140 g of a solution containing an emulsifier to the silicone-modified polioxydethylene (SF1540, Momentive ®) in the concentration dimethicone 3% m / m. Another solution was prepared by solubilizing 1.2 g chitosan and 0.6 g NaCl in 51 g of deionized water. The aqueous chitosan solution was emulsified in the hydrophilic phase not under mechanical agitation of 1000 rpm. After this emulsification, the mixture was subjected to high pressure homogenization with five cycles at 900 bar pressure. Finally, the emulsion was brought to a jacketed glass reactor to effect the removal of water by distillation under vacuum of 280 mmHg. The removal of water was carried out for 3 to 5 hours at 50 ° C. NCs obtained in this example were characterized as the average particle diameter (DP), polydispersity index (PI), residual water content (TA), viscosity (Target), turbidimetry dynamic profile morphology and size reduction as a function of water content as shown in Table 3.

**Table 3: Results obtained with the product produced according to the experimental conditions of Example 3.**

| **DP (nm)** | | **IP** | | **TA (%)** | | **Visc** |
|---|---|---|---|---|---|---|
| 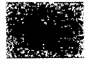 | ***5h*** | 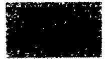 | ***5h*** | 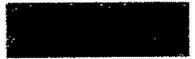 | ***5h*** | **(cP)** |
| 510 | 505 | 0,053 | 0,114 | 5,965 ± 0,427 | 0,189 ± 0,039 | 130 |

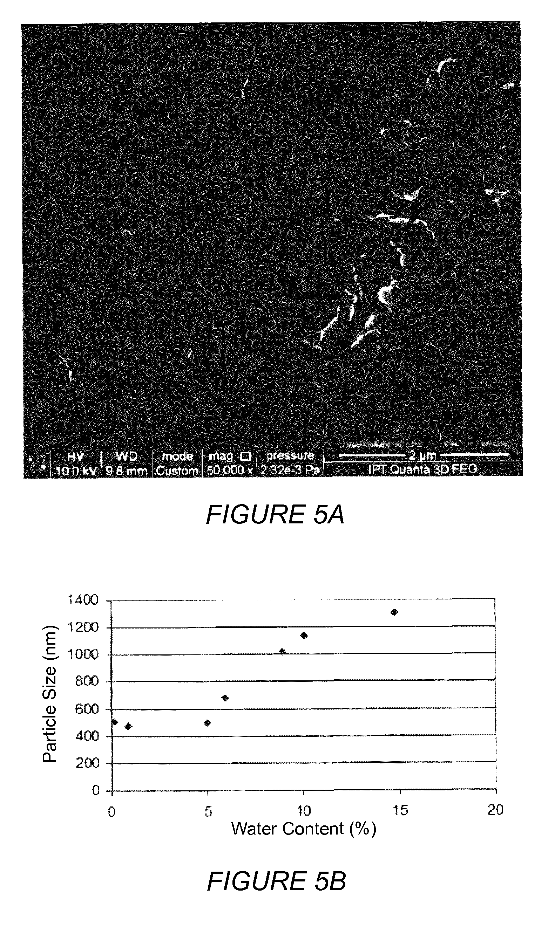

The characterization of chitosan-based NCs with respect to particle diameter shows a system to obtain nanometer-scale, low polydispersity index and water content lower than 1, 0% to 5 hours of extraction. The system obtained has characteristic of high fluidity owing to low viscosity. In this example also observed the formation of spherical nanocarrier structures, with regular surface, as shown in Figure 5A.

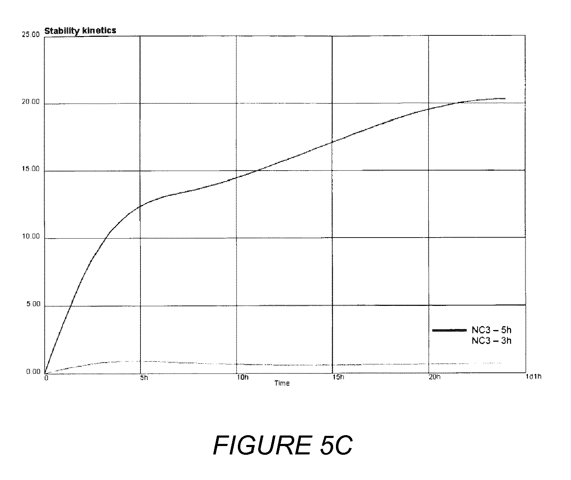

This confirms the possibility of modulation of particle size as a function of the water content present in nanocarriers, variance shows that even a level of nanometer scale, does not result in major reductions in size subsequently, as can be seen in Figure 5B. This modulation is associated yet nanocarriers physical stability of the suspension, which with 3 hours of extraction process is kinetically stable and 5 hours have reduced water content, it maintains the property nanometer, but exhibits phase separation, although this is easily redispersed. Figure 5C shows the profiles of physical stability of this product with 3 and 5 hours of dehumidification process, and Table 4 shows the levels of dynamic stability obtained by turbidimetry, in which case the lower the index, the greater the physical stability of the system.

**Table 4 - stability indices relating to Example 3 obtained by dynamic turbidimetry**

| Sample # | Stabilization index |
|---|---|
| NC3 - 3h | 0.69 |
| NC3 - 5h | 10.88 |

### EXAMPLE 4: Obtaining CN starch-based binder containing sodium salicylate as an active hydrophilic model

The NC obtaining the starch-based, containing an active model (sodium salicylate - SANA) was performed in a 500 mL beaker was prepared where 140 g of a solution containing an emulsifier to the silicone-modified polioxydethylene (SF1540, Momentive ®) in the concentration dimethicone 3% m / m. Another solution was prepared by dissolution of 9 g of starch and 0.6 g NaCl and 2 g of sodium salicylate in 49 g of deionized water. The aqueous starch and active was not hydrophilic phase emulsified under mechanical agitation of 1000 rpm. After this emulsification, the mixture was subjected to high pressure homogenization through five cycles at 900 bar pressure. Finally, the emulsion was brought to a jacketed glass reactor to effect the removal of water by distillation under vacuum of 280 mmHg. The removal of water was carried out for 3 and 5 hours of extraction process at a temperature of 50 ° C. NCs obtained in this example were characterized as the average particle diameter (DP), polydispersity index (PI), residual water content (TA), viscosity (Target) encapsulation efficiency (EE), turbidimetry dynamics and morphology, which Results are shown in Table 5.

**Table 5: Results obtained with the product produced according to the experimental conditions of Example 4.**

| **DP (nm)** | | **IP** | | **TA (%)** | | **Visc** | **EE** |
|---|---|---|---|---|---|---|---|
| 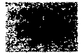 | ***5h*** | 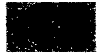 | ***5h*** | 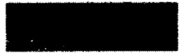 | ***5h*** | **(cP)** | **(%)** |
| 143 | 116 | 0,085 | 0,052 | 5,694 ± 0,148 | 0,165 ± 0,01 | 150,7 | 93,70 |

The DP data, shown in Table 4, confirm the nanometer scale of the NCs starch-based embedded with Sana, and are in the same size range of NCs starch without active (Example 1), indicating that the presence of the molecule not altering the size characteristics of the nanocarriers. Nevertheless, the polydispersity index is also low, and the water content in the process times 3 and 5 hours dehumidification also reproduce those of NCs without active (EXAMPLE 1), being reduced to values below 1, 0 to 5% hours of extraction. The system has also generated a fluid, confirmed by low viscosity. With the product obtained in this example, the encapsulation efficiency of the remedy the starch matrix showed a value of 93.70%, which demonstrates the feasibility of using the NCs in the encapsulation of hydrophilic active.

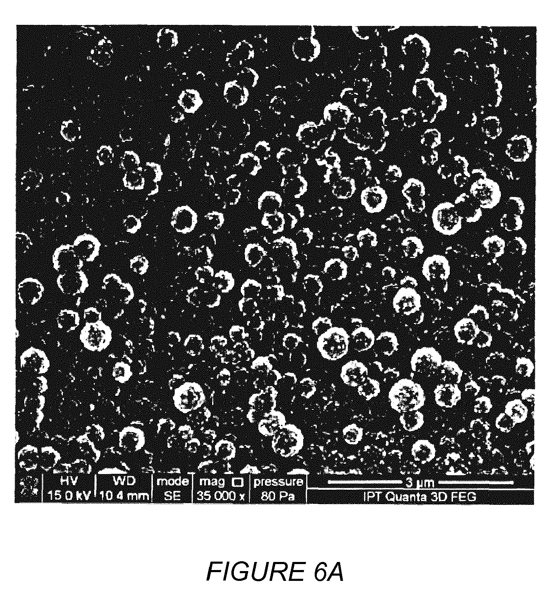

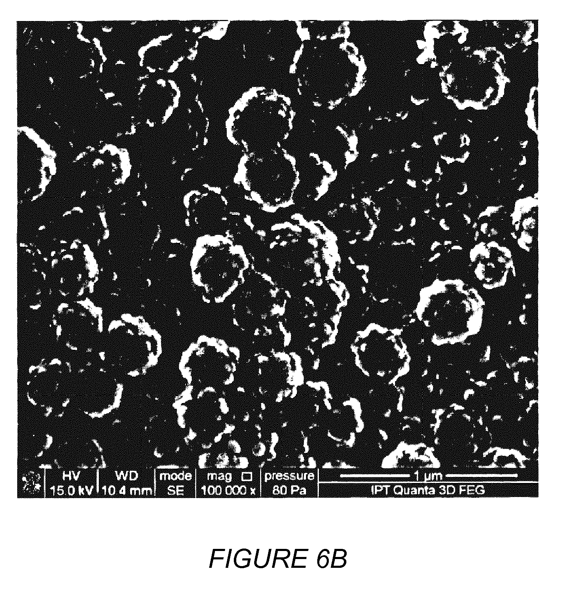

It is observed in Figures 6A and 6B that the NCs consisting of starch and Sana containing as active model showed an irregular surface with multiple protrusions on the surface of the particles, like granules active. This morphology shows that the asset is probably encapsulated distributed in the polymer matrix, with preferential location in the outermost portion of the matrix. It is assumed that during removal of the internal phase through the vacuum extraction process SANA, which has high solubility in water, migrate to the surface of the particles and becomes more trapped in the outermost layer, solidifying the water content final (less than 1, 0%) and forming small beads visible.

### EXAMPLE 5: Obtaining NC-based polyvinylpyrrolidone containing sodium salicylate as active hydrophilic model

Obtaining the CN based on PVP containing an active model (sodium salicylate - SANA) was performed in a 500 mL beaker was prepared where 140 g of a solution containing an emulsifier to the silicone-modified polioxydethylene (SF1540, Momentive ®) in the concentration dimethicone 3% m / m. Another solution was prepared by dissolution of 9 g of PVP, 0.6 g NaCl and 2 g of sodium salicylate in 49 g of deionized water. The aqueous solution of PVP and active was emulsified in the hydrophilic phase not under mechanical agitation of 1000 rpm. After this emulsification, the mixture was subjected to high pressure homogenization with five cycles pressure of 900 bar. Finally, the emulsion was brought to a jacketed glass reactor to effect the removal of water by distillation under vacuum of 280 mmHg. The removal of water was carried out for 3 to 5 hours (NC5 - NC5-3h and 5h) at a temperature of 50 ° C. NCs obtained in this example were characterized as the average particle diameter (DP), polydispersity index (PI), residual water content (TA), viscosity (Target), turbidimetry and dynamic encapsulation efficiency (EE), and the results are shown in Table 6.

**Table 6: Results obtained with the product produced according to the experimental conditions of Example 5.**

| **DP (nm)** | | **IP** | | **TA (%)** | | **Visc** | **EE** |
|---|---|---|---|---|---|---|---|
| 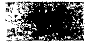 | ***5h*** | 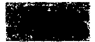 | ***5h*** | 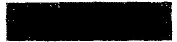 | ***5h*** | **(cP)** | **(%)** |
| 126,1 | 202,0 | 0,328 | 0,541 | 5,940 ± 0,376 | 0,291 ± 0,028 | 104,7 | 84,60 |

Similarly to the previous examples, the NCs-based PVP containing as active SANA DP model presented at the nanoscale, low polydispersity, reducing water content up to the time of extraction process, with values below 1, 0% to 5 hours Low viscosity process and indicating the fluidity of the system.

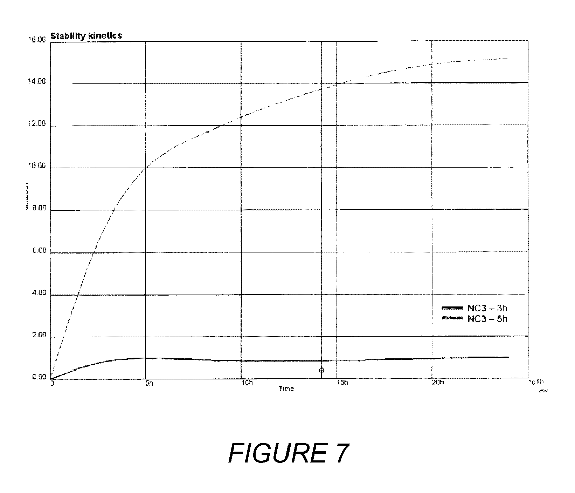

Figure 7 shows the profiles of physical stability of this product with 3 and 5 hours of water extraction process as well as the stability indices obtained by varying the backscattering as a function of time as shown in Table 7 (data obtained by turbidimetry dynamics). NCs with 3 hours show a much lower stability index (greater stability) compared to those from 5-hour extraction process, indicating a change in the physical stability of the system with the water content present.

**Table 7 - stability indices relating to Example 5, obtained by dynamic turbidimetry**

| Sample # | Stabilization index |
|---|---|
| NC3 - 3h | 0.78 |
| NC3 - 5h | 8.95 |

The encapsulation efficiency also resulted in a higher value (84.60%) showing the performance of the array nanocarriers in the incorporation of hydrophilic molecules.

### EXAMPLE 6: Obtaining NC-based starch containing hydrophilic active as a cyanocobalamin model

The NC obtaining starch-based, containing an active model (cyanocobalamin) was performed in a 500 mL beaker was prepared where 140 g of a solution containing an emulsifier to the silicone-modified polioxydethylene (SF1540, Momentive ®) in dimethicone concentration 3% m / m. Another solution was prepared by dissolution of 8.1 g starch, 0.6 g NaCl and 0.9 g of sodium salicylate in 51 g of deionized water. The aqueous starch and active was not hydrophilic phase emulsified under mechanical agitation of 1000 rpm. After this emulsification, the mixture was subjected to high pressure homogenization with five cycles at 900 bar pressure. Finally, the emulsion was brought to a jacketed glass reactor to effect the removal of water by distillation under vacuum of 280 mmHg. The removal of water was carried out for 3 to 5 hours at 50 ° C. The NCs obtained in this example were characterized as the average particle diameter (DP), polydispersity index (PI), residual water content (TA), refractive index (RI), viscosity (Target), turbidimetry dynamics and morphology, as shown in Table 8.

**Table 8: Results obtained with the product produced according to the experimental conditions of Example 6.**

| **DP (nm)** | | **IP** | | **TA (%)** | | **Visc** | **EE** |
|---|---|---|---|---|---|---|---|
| 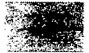 | ***5h*** | 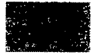 | ***5h*** | 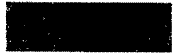 | ***5h*** | **(cP)** | **(%)** |
| 288,1 | 316,6 | 0,089 | 0,133 | 6,153 ± 0,03 | 0,258 ± 0,055 | 102,5 | 88,68 |

In this example demonstrates the incorporation of a hydrophilic active use food, pharmaceutical and veterinary NCs in starch based. The product obtained in the experimental conditions of this example DP presented at the nanoscale, low polydispersity, reducing water content with time dehumidification process, with values below 1, 0% to 5 hour process and low viscosity indicating the fluidity of the system.

The encapsulation efficiency also resulted in a large value (approximately 89%) demonstrating the performance of the array nanocarriers in the incorporation of hydrophilic molecules.

### EXAMPLE 7: Profile of controlled release of active models of NCs

The profile of controlled release of active models of NCs, relating to Examples 4 and 6 was monitored in a 48 hours period, by measuring the UV absorption in the wavelengths of 232 nm to 301 nm to cyanocobalamin and sodium salicylate.

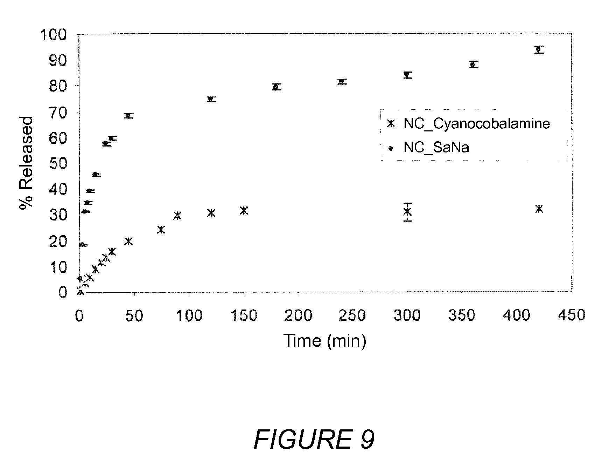

A mass of about 150 g of the formulation was applied to a membrane and immersed in closed flasks, to which were added 100.0 ml of water. The system was kept under gentle stirring (50 rpm) and heated to 37 ° C. There were collected 3.0 mL aliquots at intervals of predetermined time and the concentrations monitored by UV absorption. The percentage of active released was plotted against time (in hours) with their standard deviations. The release assay was performed in triplicate and reading of absorption measurements in quintuplicate, as can be seen in Figure 9.

It is observed that the NCs containing sodium salicylate showed a release profile faster than those containing cyanocobalamin. These differences in the release profile may be attributed to differences in molecular structure and solubility of the molecules (hydrophilic active) in water, besides the interaction that each has with the polymeric matrix.

### REFERENCES

1. ARRUEBO, M., FERNANDEZ PACHECO, R., IBARRA, MR; SANTAMARIA, J. Magnetic nanoparticles for drug delivery, Nano Today, v. 2, p. 22-32, June 2007.
2. GUERY, J. Double emulsions crystallizable: Stability, Encapsulation and release. 2006. 177 f. PhD - UNIVERSITY PARIS VI - Graduate School of Physics and Chemistry of Materials, 2006.
3. JENNING, V.; SCHAFER-KORTING, M.; GOHLA, S. Vitamin A-loaded solid lipid nanoparticles for topical use drug release properties, Journal of Controlled Release, v. 66, p. 1 15-126, May 2000.
4. KR 100868724 - "Method for Preparing Self-Aggregating Nanocarrier Particles Having Temperature Depending Property"
5. LEE, S.; KURIHARA-BERGSTROM, T.; KIM, S. Nonaqueous drug permeation through synthetic membranes. Journal of Controlled Release, v. 5, p. 253-262, 1988.
6. LIM, S. J.; KIM, C. K. Formulation parameters determining the physicochemical characteristics of solid lipid nanoparticles loaded with all-trans retinoic acid, International Journal of Pharmaceutics, v. 243, p. 135-146, Aug 2002.
7. LIMA, E.; KEDOR-HACKMANN, E. Therapeutic utility of lipossomes , Revista Brasileira de Medicina, v. 51 , p. 585-590, May 1994.
8. LIPPACHER, A.; MULLER, R. H.; MADER, K. Preparation of semisolid drug carriers for topical application based on solid lipid nanoparticles, International Journal of Pharmaceutics, v. 214, p. 9-12, Feb.2001.
9. MAIA, C. S.; MEHNERT, W.; SCHAFER-KORTING, M. Solid lipid nanoparticles as drug carriers for topical glucocorticoids, International Journal of Pharmaceutics, v. 196, p. 165-167, Mar.2000.
10. MEHNERT, W.; MADER, K. Solid lipid nanoparticles - Production, characterization and applications, Advanced Drug Delivery Reviews, v. 47, p. 165-196, Apr.2001.
11. MISHRA B, BP BHAVESH, AND T SANJAY. Colloidal nanocarriers: a review on formulation technology, types and applications toward targeted drug delivery. Nanomedicine: Nanotechnology, Biology and Medicine, 18 May 2009.
12. MOU, D. S.; CHEN, H. B.; DU, D. R.; MAO, C. W.; WAN, H. L; XU, H. B.; YANG, X. L. Hydrogel-thickened nanoemulsion system for topical delivery of lipophilic drugs, International Journal of Pharmaceutics, v. 353, p. 270-276, Apr.2008.
13. MUHLEN, A.; SCHWARZ, C; MEHNERT, W. Solid lipid nanoparticles (SLN) for controlled drug delivery - Drug release and release mechanism, European Journal of Pharmaceutics and Biopharmaceutics, v. 45, p. 149-155, Mar 1998.
14. MULLER, R. H.; MADER, K.; GOHLA, S. Solid lipid nanoparticles (SLN) for controlled drug delivery - a review of the state of the art, European Journal of Pharmaceutics and Biopharmaceutics, v. 50, p. 161-177, July 2000.
15. MULLER, R. H.; RADTKE, M.; WISSING, S. A. Solid lipid nanoparticles (SLN) and nanostructured lipid carriers (NLC) in cosmetic and dermatological preparations, Advanced Drug Delivery Reviews, v. 54, p. S131-S155, Nov 2002.
16. MULLER-GOYMANN, C. C. Physicochernical characterization of colloidal drug delivery systems such as reverse micelles, vesicles, liquid crystals and nanoparticles for topical administration, European Journal of Pharmaceutics and Biopharmaceutics, v. 58, p. 343-356, Sept 2004.
17. AGARWAL, R. C; KANT, S.; SINGH, P. N.; MAITI, P.; PANDIT, J. K. Polymeric nanoparticulate system: A potential approach for ocular drug delivery, Journal of Controlled Release, v. 136, p. 2-13, May2009.
18. RUENRAROENGSAK, P.; COOK, J. M.; FLORENCE, A. T. Nanosystem drug targeting: Facing up to complex realities. Journal of Controlled Release , v.141 , n.p.265-76, 2010.
19. SAKULKU, U.; NUCHUCHUA, O.; UAWONGYART, N.; PUTTIPIPATKHACHORN, S.; SOOTTITANTAWAT, A.; RUKTANONCHAI, U. Characterization and mosquito repellent activity of citronella oil nanoemulsion, International Journal of Pharmaceutics, v. 372, p. 105-1 1 1 , May 2009.
20. SUITTHIMEATHEGORN, O.; JAITELY, V.; FLORENCE, A. T. Novel anhydrous emulsions: Formulation as controlled release vehicles, International Journal of Pharmaceutics, v. 298, p. 367-371 , July 2005.
21. TADROS, T.; IZQUIERDO, R.; ESQUENA, J.; SOLANS, C. Formation and stability of nano-emulsions, Advances in Colloid and Interface Science, v. 108-09, p. 303-318, May 2004.
22. TORCHILIN, V. Multifunctional and stimuli-sensitive pharmaceutical nanocarriers, European Journal of Pharmaceutics and Biopharmaceutics, v. 71 , p. 431-444, Mar 2009.
23. WISSING, S. A.; MULLER, R. H. A novel sunscreen system based on tocopherol acetate incorporated into solid lipid nanoparticles, International Journal of Cosmetic Science, v. 23, p. 233-243, Aug 2001.
24. WISSING, S. A.; MULLER, R. H. Solid lipid nanoparticles as carrier for sunscreens: in vitro release and in vivo skin penetration, Journal of Controlled Release, v. 8 , p. 225-233, June 2002a.
25. WISSING, S. A.; MULLER, R. H. The development of an improved carrier system for sunscreen formulations based on crystalline lipid nanoparticles, International Journal of Pharmaceutics, v. 242, p. 373-375, Aug 2002b.
26. WO/2007/041206 - "Drug Delivery Nanocarriers Targeted by Landscape Phage"
27. WO/2009/123768 - "Nanocarrier and Nanogel Compositions",
28. WO/2009/123934 - "Branched Multifunctional Nanoparticle Conjugates and their Use"
29. WU, H. L; RAMACHANDRAN, C; BIELINSKA, A. U.; KINGZETT, K.; SUN, R.; WEINER, N. D.; ROESSLER, B. J. Topical transfection using plasmid DNA in a water-in-oil nanoemulsion, International Journal of Pharmaceutics, v. 221 , p. 23-34, June2001a.
30. WU, H. L; RAMACHANDRAN, C; WEINER, N. D.; ROESSLER, B. J. Topical transport of hydrophilic compounds using water-in-oil nanoemulsions, International Journal of Pharmaceutics, v. 220, p. 63-75, June 2001 b.
31. YILMAZ, E.; BORCHERT, H. H. Design of a phytosphingosine-containing, positively-charged nanoemulsion as a colloidal carrier system for dermal application of ceramides, European Journal of Pharmaceutics and Biopharmaceutics, v. 60, p. 91-98, May 2005.

## Claims

1. Colloid nanocarriers for hydrophilic actives, **characterized in that** they comprise structures formed by hydrophilic polymers containing hydrophilic actives, surrounded by a non-hydrophilic phase and surfactants being attracted by the components, thus forming a reverse emulsion.

2. Colloid nanocarriers for hydrophilic actives, according to claim 1, **characterized in that** the hydrophilic polymers are polysaccharides, proteins of natural origin, chitosan, arabic gum, xanthan gum, guar gum, carrageenan gum, cashew gum, tara gum, tragacanth gum, karaya gum, gati gum or derivatives thereof, cellulose, carboxymethyl cellulose, carboxyethyl cellulose, polyvinylpyrrolidone (PVP), polyacrylates, polymethacrylates and polyacrylamides or polivinilcaprolactamas.

3. Colloid nanocarriers for hydrophilic actives, according to claim 1, **characterized in that** the non-hydrophilic phase comprises lipophilic or silophilic liquids.

4. Colloid nanocarriers for hydrophilic actives, according to claim 3, **characterized in that** the non-hydrophilic phase is dimethicone.

5. Colloid nanocarriers for hydrophilic actives, according to claim 1, **characterized in that** the co-stabilizer is an inorganic salt.

6. Colloid nanocarriers for hydrophilic actives, according to claim 5, **characterized in that** the co-stabilizer is a mono-or bivalent chloride.

7. Colloid nanocarriers for hydrophilic actives, according to claim 6, **characterized in that** the co-stabilizer is sodium chloride.

8. Production process for the production of nanocarriers for hydrophilic actives, **characterized in that** it comprises three steps: the first step being the formation of a pre-emulsion by dispersing the internal phase in the external phase, the internal phase being composed by a polymer and an aqueous solution containing an inorganic salt and the water soluble hydrophilic active while the external phase comprises the hydrophilic component and the non-specific emulsifier; a second step of nanoemulsification consisting in homogenizing the pre-emulsion formed in the said first step in a mixture system of high energy breakdown and a third step of 3 extraction of the aqueous phase forming the internal colloidal nanocarriers.

9. Production process, according to claim 8, **characterized in that** the first step is performed at a temperature from 10 to 100 ° C, stirring from 100 to 22,000 rpm and under atmospheric pressure.

10. Production process, according to claim 9, **characterized in that** the first step is carried out at a temperature of 25.0 ° C and with stirring at 1000 rpm.

11. Production process, according to claim 8, **characterized in that** the second step is carried out at a temperature from 10 to 100 ° C under a pressure of at least 10 bar and using a minimum of one cycle by varying the pressure of homogenization.

12. Production process, according to claim 11, **characterized in that** the second step is carried out at a temperature of 25 ° C and under a pressure of 900 Bar for up to 20 cycles.

13. Production process, according to claim 8, **characterized in that** the third step is performed at a temperature of 20 to 50 ° C and under a pressure from 760 mm Hg to 10⁷ mmHg for at least 15 minutes.

14. Production process, according to claim 8, **characterized in that** the third step is carried out at a temperature of 50 ° C and under pressure of 280 mmHg for 5 hours.
